Europäisches Patentamt

European Patent Office

Office européen des brevets

(11) Veröffentlichungsnummer: **0 071 076**
B1

(12) **EUROPÄISCHE PATENTSCHRIFT**

(45) Veröffentlichungstag der Patentschrift:
02.05.85

(51) Int. Cl.⁴: **C 12 Q 1/34,** C 12 Q 1/42,
C 12 Q 1/48

(21) Anmeldenummer: **82106278.3**

(22) Anmeldetag: **14.07.82**

(54) Verfahren zur Bestimmung von Lecithin sowie Reagens zur Durchführung dieses Verfahrens.

(30) Priorität: **18.07.81 DE 3128480**

(43) Veröffentlichungstag der Anmeldung:
**09.02.83 Patentblatt 83/6**

(45) Bekanntmachung des Hinweises auf die Patenterteilung:
**02.05.85 Patentblatt 85/18**

(84) Benannte Vertragsstaaten:
**AT BE CH DE FR GB IT LI LU NL SE**

(56) Entgegenhaltungen:
**DE - A - 2 737 513**
**GB - A - 1 555 173**
**US - A - 4 168 203**

*Geburtshilfe u. Frauenheilkunde 39 (1979), S. 849-856*
**Römpps Chemie-Lexikon (7. Auflage 1977) - 3602**
**(Tino...)**

**Die Akte enthält technische Angaben, die nach dem Eingang der Anmeldung eingereicht wurden und die nicht in dieser Patentschrift enthalten sind.**

(73) Patentinhaber: **Boehringer Mannheim GmbH,**
**Sandhoferstrasse 116, D-6800 Mannheim 31 (DE)**

(72) Erfinder: **Becker, Jürgen, Loisachstrasse 27,**
**D-8122 Penzberg-Maxkron (DE)**
Erfinder: **Beinstingl, Gerhard, Fischerweg 2,**
**D-8121 Wielenbach (DE)**
Erfinder: **Beutler, Hans-Otto, Dr.rer.nat.,**
**Zugspitzstrasse 28, D-8132 Tutzing (DE)**

## Beschreibung

Gegenstand der Erfindung ist ein Verfahren zur Bestimmung von Lecithin in einer Probe durch enzymatische Spaltung des Lecithins mit Hilfe von Phospholipase-C und alkalischer Phosphatase zu Cholin, Umsetzung des Cholins mit Hilfe von Cholinkinase in Gegenwart von Adenosintriphosphat zu Phosphorylcholin und anschliessendem Nachweis des entstandenen Adenosindiphosphats nach bekannten Methoden, wobei die Spaltung des Lecithins in Gegenwart eines Borat/Borax-Puffers und eines nicht-ionischen Detergens auf der Basis eines Kondensationsproduktes von Polyethylenglykol mit Isooctylphenol durchgeführt wird.

Eine Bestimmung des Lecithins wird beispielsweise bei der Untersuchung von Lebensmitteln durchgeführt. Lecithin kommt vor allem im Eidotter vor. Der Lecithingehalt des Eidotters unterliegt nur geringen Schwankungsbreiten, so dass darin ein Mass für den Eigehalt einer Lebensmittelprobe gesehen werden kann.

Besondere Bedeutung hat die Bestimmung des Lecithingehalts im Fruchtwasser. Damit lässt sich die fetale Lungenreife bereits vor der Geburt weitgehend feststellen und das mögliche Risiko eines Atemnotsyndroms für das Neugeborene abschätzen. Das Atemnotsyndrom ist eine der wichtigsten Komplikationen, die bei Frühgeborenen nach der Geburt auftreten können. Es wird durch einen Mangel an Surfactant hervorgerufen, der bei reifen Neugeborenen als dünner Film die Lungenalveolen auskleidet und die Aufgabe hat, die Oberflächenspannung an der Grenzfläche Luft/Flüssigkeit in den Alveolen zu senken und dadurch die Stabilität der Alveolen zu gewährleisten. Der Surfactant besteht aus Proteinen, Kohlenhydraten und Lipoiden, wobei den Phospholipiden, beispielsweise Dipalmitoyllecithin, Phosphatidylglycerol und Sphingomyelin, die entscheidenden oberflächenaktiven Eigenschaften zukommen.

Der Lecithingehalt im Fruchtwasser bewegt sich während der Schwangerschaft lange Zeit zwischen 1 und 2 mg/dl, steigt in der 35. bis 37. Woche auf 5-10 mg/dl und liegt in der 40. Woche meist bei einem Wert über 10 mg/dl. Der steile Anstieg der Lecithinkonzentration zwischen der 35. und 37. Woche der Schwangerschaft kann als charakteristisches Mass für das Vorliegen der fetalen Lungenreife herangezogen werden.

Durch die Atembewegungen des Feten kommt es zu einem ständigen Austausch der Alveolarflüssigkeit mit dem Fruchtwasser, so dass die Möglichkeit besteht, sich durch die Messung des Phospholipidgehalts im Fruchtwasser ein Bild vom Surfactantgehalt in den fetalen Alveolen zu machen und dadurch bereits vor der Geburt eine Aussage über den Reifezustand der Lungen zu erhalten. Die Bestimmung des Lecithins im Fruchtwasser ist daher von entscheidender Bedeutung für die Schwangerschaftsdiagnostik, insbesondere im Hinblick auf eine frühzeitige Terminierung einer Risikoschwangerschaft wegen eines drohenden Atemnotsyndroms.

Für die Bestimmung des Lecithins im Fruchtwasser stehen zahlreiche Methoden zur Verfügung, die sich einerseits durch den unterschiedlichen Arbeitsaufwand und andererseits durch die Genauigkeit der Vorhersage teilweise beträchtlich unterscheiden.

Aus Geburtshilfe und Frauenheilkunde 39 (1979) S. 849-856 ist eine enzymatische Lecithinbestimmung in Fruchtwasser bekannt, die leichter durchführbar ist als die nicht-enzymatischen Methoden und diesen an Genauigkeit und Praktikabilität gleichwertig, oft sogar überlegen ist. Das Lecithin wird bei dieser Bestimmungsmethode mit Hilfe von Phospholipase-C in einer Glycinpuffer enthaltenden Lösung zu Phosphorylcholin gespalten, das durch alkalische Phosphatase weiter zu Cholin abgebaut wird. Letzteres wird in Gegenwart von Adenosintriphosphat mit Hilfe von Cholinkinase wieder zu Phosphorylcholin unter Bildung von Adenosindiphosphat umgesetzt. Das gebildete Adenosindiphosphat wird über eine an sich bekannte enzymatische Reaktionskette photometrisch gemessen. Dieses Nachweisverfahren kann durch die folgenden Reaktionsgleichungen beschrieben werden:

$$(1) \quad \text{Lecithin} + H_2O \xrightarrow{\text{Phospholipase-C}} \text{1,2-Diglycerid} + \text{Phosphorylcholin}$$

$$(2) \quad \text{Phosphorylcholin} + H_2O \xrightarrow{\text{AP}} \text{Cholin} + PO_4^{3-}$$

$$(3) \quad \text{Cholin} + \text{ATP} \xrightarrow{\text{Cholinkinase}} \text{Phosphorylcholin} + \text{ADP}$$

$$(4) \quad \text{ADP} + \text{PEP} \xrightarrow{\text{PK}} \text{ATP} + \text{Pyruvat}$$

$$(5) \quad \text{Pyruvat} + \text{NADH} + H^+ \xrightarrow{\text{LDH}} \text{Lactat} + \text{NAD}^+$$

Die benutzten Abkürzungen bedeuten im Einzelnen:

| | |
|---|---|
| AP | = alkalische Phosphatase |
| ATP | = Adenosin-5′-triphosphat |
| ADP | = Adenosin-5′-diphosphat |
| PEP | = Phosphoenolpyruvat |
| PK | = Pyruvatkinase |
| NADH/NAD$^+$ | = Nicotinamid-adenin-dinucleotid, reduzierte/oxidierte Form |
| LDH | = Lactatdehydrogenase |

Die Beurteilung eines drohenden Atemnotsyndroms ist zwar durch das enzymatische Verfahren zur Bestimmung von Lecithin in Fruchtwasser wesentlich vereinfacht worden. Es weist jedoch einen erheblichen Nachteil auf: In einer beachtlichen Anzahl von Proben treten bei der Messung Störungen auf, die von einer starken Trübung der Messlösung herrühren und zuweilen eine Lecithinbestimmung völlig unmöglich machen. Der Grund liegt in der hohen Trübung des Fruchtwassers, das mit einem relativ grossen Probevolumen in die Küvette eingesetzt wird und daher auch im Test eine starke, nicht mehr messbare Trübung verursachen kann. Es werden zu hohe Anfangsextinktionen gemessen, die zu einer hohen Fehlerquote und zu einer mangelhaften Präzision der Messung führen. Ferner werden bei der Messung solcher Proben erhebliche Abweichungen von der Linearität der Messung gefunden.

Aufgabe der Erfindung war es nun, ein Verfahren bereitzustellen, das die oben erwähnten Nachteile nicht mehr aufweist.

Gelöst wurde diese Aufgabe durch das erfindungsgemässe Verfahren, das von dem Verfahren gemäss dem Stand der Technik dadurch abweicht, dass die Spaltung des Lecithins mit Phospholipase-C und alkalischer Phosphatase nicht in einem Glycin-Puffer, sondern in einem Borat/Borax-Puffer in Gegenwart eines nicht-ionischen Detergens auf der Basis eines Kondensationsproduktes von Polyethylenglykol mit Isooctylphenol durchgeführt wird.

Grundsätzlich ist eine Klärung der zu messenden Lösung durch viele Detergentien erreichbar. Doch zeigen die meisten Detergentien eine mehr oder weniger stark ausgeprägte Hemmwirkung auf die enzymatisch ablaufenden Prozesse, die zu falschen Messergebnissen führen. Andererseits laufen enzymatische Reaktionen in befriedigender Weise nur in einem relativ engen pH-Bereich ab. Im Falle der Lecithinbestimmung in einer Probe ist ein pH-Bereich von 7,0-8,5 optimal. Darüber hinaus hat sich gezeigt, dass für das komplexe System der enzymatischen Reaktionen, die für die Lecithinbestimmung erforderlich sind, nur wenige Puffersysteme in Frage kommen.

Es hat sich überraschenderweise gezeigt, dass optimale Messbedingungen für eine störungsfreie Bestimmung von Lecithin in einer Probelösung erreicht werden können, wenn bei der Spaltung des Lecithins ein Borat/Borax-Puffer und ein nicht-ionisches Detergens auf der Basis eines Kondensationsproduktes von Polyethylenglykol mit Isooctylphenol verwendet werden.

Gegenstand der Erfindung ist daher ein Verfahren zur Bestimmung von Lecithin in einer Probe, bei dem zunächst Lecithin mit Hilfe von Phospholipase-C und alkalischer Phosphatase zu Cholin gespalten, danach das entstandene Cholin in Gegenwart von Adenosintriphosphat mit Hilfe von Cholinkinase wieder zu Phosphorylcholin umgesetzt und das dabei gebildete Adenosindiphosphat nach bekannten Methoden gemessen wird, dadurch gekennzeichnet, dass die Schaltung des Lecithins in Gegenwart eines Borat/Borax-Puffers und in Gegenwart eines nicht-ionischen Detergens auf der Basis eines Kondensationsproduktes von Polyethylenglykol mit Isooctylphenol durchgeführt wird.

Gemäss einer bevorzugten Ausführungsform des erfindungsgemässen Verfahrens wird zunächst die Probe — beispielsweise Fruchtwasser, gegebenenfalls mit Wasser verdünnt — mit dem Borat/Borax-Puffer, einem Magnesiumsalz, dem Detergens, mit Phospholipase-C und alkalischer Phosphatase vermischt und bei Raumtemperatur oder leicht erhöhter Temperatur inkubiert. Die Inkubationszeit beträgt je nach der gewählten Temperatur vorzugsweise 10 bis 60 Minuten. Danach wird kurze Zeit auf 95 bis 100°C, beispielsweise in einem Wasserbad, erhitzt. Nach dem Abkühlen des Gemisches wird abzentrifugiert und der Überstand mit NADH, ATP, Phosphoenolpyruvat, Glukose, Pyruvatkinase und Lactatdehydrogenase versetzt. Nach kurzer Wartezeit (ca. 5 Minuten) wird die Extinktion $E_1$ gemessen. Anschliessend wird eine Cholinkinase-Lösung zugegeben, um die Reaktionskette zum Nachweise des aus Lecithin freigesetzten Cholins in Gang zu setzen. Ist die Reaktion abgeschlossen, wird die Extinktion $E_2$ gemessen. Aus der Extinktionsdifferenz lässt sich der Lecithingehalt der Probe ermitteln. Es empfiehlt sich, neben der eigentlichen Bestimmung der Probe einen Reagentienleerwert zu messen.

Es ist zweckmässig, die für das erfindungsgemässe Verfahren benötigten Enzyme und Hilfsstoffe zu bestimmten Lösungen bzw. Suspensionen zusammenzufassen.

So wird vorzugsweise der Borat/Borax-Puffer zusammen mit dem Detergens und dem Magnesiumsalz in Form einer «Pufferlösung» eingesetzt. Vorteilhafterweise wird ein Borat/Borax-Puffer mit pH 7,0 bis 8,5 in einer Konzentration von 0,05-0,5 mol/l Pufferlösung, vorzugsweise von 0,07-0,25 mol/l Pufferlösung, eingesetzt. Ganz besonders bevorzugt ist ein Borat/Borax-Puffer vom pH-Wert 7,8-8,2, der in einer Konzentration von 0,1-0,2 mol/l Pufferlösung bei der Bestimmung benutzt wird. Das nichtionische Detergens auf der Basis eines Kondensationsproduktes von Polyethylenglykol mit Isooctylphenol wird in einer Konzentration von 0,5-5 g/l, vorzugsweise 1,0-3,0 g/l, der Pufferlösung zugesetzt. Als erfindungsgemässes Detergens hat sich besonders das Handelsprodukt Tinovetin JU® der Fa. Ciba Geigy bewährt.

Die Phospholipase-C und die alkalische Phosphatase werden zusammen als «Enzymsuspension A» verwendet, wobei die Konzentration an Phospholipase-C 40-200 U/ml, vorzugsweise 100 U/ml, und die Konzentration an alkalischer Phosphatase 20 bis 200 U/ml, vorzugsweise 80 U/ml, betragen.

NADH, ATP, Phosphoenolpyruvat und Glukose werden in vorteilhafter Weise als «Coenzymlösung» zusammengefasst. Die einzelnen Konzentrationen in dieser Lösung betragen 2,5-6,0 mmol/l, vorzugsweise 4,0 mmol/l NADH, 10-30 mmol/l, vorzugsweise 20 mmol/l ATP, 3-10 mmol/l, vorzugsweise 7 mmol/l Phosphoenolpyruvat und 30-100 mmol/l, vorzugsweise 50-60 mmol/l Glukose.

Pyruvatkinase und Lactatdehydrogenase werden zusammen als «Enzymsuspension B» zugegeben, wobei die Konzentrationen an Pyruvatkinase und an Lactatdehydrogenase jeweils 150-1000 U/ml, vorzugsweise 300 U/ml betragen.

Cholinkinase wird vorzugsweise in Form einer getrennten Lösung mit einem Gehalt an 2-10 U/ml, vorzugsweise 2,5 U/ml verwendet.

Gegenstand der Erfindung ist ferner ein Reagens zur Lecithinbestimmung, das aus folgenden Bestandteilen besteht.

a) einer Pufferlösung mit
    0,05-0,5 mol/l Borat/Borax-Puffer, pH 7,0 bis 8,5
    2-20 mmol/l Magnesiumionen
    0,5-5,0 g/l Detergens auf der Basis eines Kondensationsproduktes von Polyethylenglykol mit Isooctylphenol

b) einer Enzymsuspension A mit
    40-200 U/ml Phospholipase-C
    20-200 U/ml alkalische Phosphatase

c) einer Coenzym-Lösung mit
    2,5-6,0 mmol/l NADH
    10-30 mmol/l ATP
    3-10 mmol/l Phosphoenolpyruvat
    30-100 mmol/l Glukose

d) einer Enzymsuspension B mit
    150-1000 U/ml Pyruvatkinase
    150-1000 U/ml Lactatdehydrogenase

e) einer Lösung mit
    2-10 U/ml Cholinkinase.

Ganz besonders bevorzugt ist ein Reagens mit folgenden Bestandteilen:

a) einer Pufferlösung mit
    0,2 mol/l Borat/Borax-Puffer, pH 7,8-8,2
    10 mmol/l Magnesiumsulfat
    2,5 g/l Tinovetin JU®

b) einer Enzymsuspension A mit
    80 U/ml Phospholipase-C
    100 U/ml alkalische Phosphatase

c) einer Coenzym-Lösung mit
    4,0 mmol/l NADH
    20 mmol/l ATP
    7 mmol/l Phosphoenolpyruvat
    56 mmol/l Glukose

d) einer Enzymsuspension B mit
    300 U/ml Pyruvatkinase
    300 U/ml Lactatdehydrogenase

e) einer Lösung mit
    2,5 U/ml Cholinkinase.

Ein weiterer Gegenstand der Erfindung ist ein Reagens zur Spaltung des Lecithins, gekennzeichnet durch einen Gehalt an

2-10 U/ml Phospholipase-C
1-10 U/ml alkalische Phosphatase
0,05-0,5 mol/l Borat/Borax-Puffer, pH 7,0-8,5
2-20 mmol/l Magnesiumionen
0,5-5,0 g/l Detergens auf der Basis eines Kondensationsproduktes von Polyethylenglykol mit Isooctylphenol.

Zur Spaltung des Lecithins ist eine Reagentienzusammensetzung ganz besonders bevorzugt, die die einzelnen Bestandteile in folgenden Konzentrationen enthält:

4 U/ml Phospholipase-C
5 U/ml alkalische Phosphatase
0,2 mol/l Borat/Borax-Puffer, pH 7,8-8,2
10 mmol/l Magnesiumsulfat
2,5 g/l Tinovetin JU®.

Das erfindungsgemässe Reagens zur Spaltung des Lecithins wird zweckmässigerweise hergestellt, indem 1,0 ml der oben beschriebenen Pufferlösung mit 50 µl der oben erwähnten Enzymsuspension A versetzt wird. Das so erhaltene Reagens wird der zu messenden Probe vorzugsweise im Verhältnis 1:1 zugesetzt.

Die Erfindung wird durch folgende Beispiele näher erläutert:

*Beispiel 1*

*Bestimmung von Lecithin in Fruchtwasser*

1 ml einer Probelösung, die ca. 0,1 mg Lecithin enthält, wird mit 1 ml einer Lösung, die 0,2 mol/l Borat/Borax-Puffer, pH 7,8-8,2, 10 mmol/lm Magnesiumsulfat und 2,5 g/l Tinovetin JU® enthält, versetzt. Zu diesem Gemisch werden 0,05 ml einer Enzymsuspension aus 80 U/ml Phospholipase-C und 100 U/ml alkalische Phosphatase gegeben. Das Gemisch wird bei 37 °C 20 Minuten stehengelassen. Danach wird 5 Minuten auf 95-100 °C erhitzt. Nach dem Abkühlenn auf Raumtemperatur werden 0,1 ml einer Lösung aus 4,0 mmol/l NADH, 20 mmol/l ATP, 7 mmol/l Phosphoenolpyruvat und 56 mmol/l Glukose sowie 0,02 ml eines Enzymgemisches aus 300 U/ml Pyruvatkinase und 300 U/ml Lactatdehydrogenase zugesetzt. Die erhaltene Lösung wird durchmischt und 10 Minuten bei Raumtemperatur stehengelassen. Die Probe wird in eine Küvette überführt und die Extinktion (E$_1$) gemessen. Die enzymatische Umsetzung wird gestartet durch Zusatz von 0,05 ml einer Cholinkinase-Lösung mit 2,5 U/ml. Nach 30 Minuten Stehenlassen bei Raumtemperatur wird die Extinktion (E$_2$) gemessen. Aus der Extinktionsdifferenz E$_1$-E$_2$ wird die Extinktionsabnahme $\triangle$E berechnet, aus der sich der Lecithingehalt der Probe nach der Formel:

$$\text{Lecithin (mg/100 ml)} = \triangle E \cdot 47,86$$

ergibt.

Zu jeder Bestimmung von Lecithin in einer Probelösung wird ein Reagentienleerwert ermittelt, der sich ergibt, wenn bei dem oben beschriebenen Bestimmungsverfahren anstelle der Probelösung 1 ml destilliertes Wasser eingesetzt wird. Der Reagentienleerwert wird vor der Berechnung des Lecithingehalts nach der o.a. Formel von der gefundenen Extinktionsabnahme $\triangle$E abgezogen.

*Beispiel 2*

*Bestimmung von Lecithin in Mayonnaise*

5 g Mayonnaise werden mit 30 ml Ethanol/Benzol-Lösung (1 + 1, v/v) und 50 ml Wasser im Scheidetrichter etwa 5 Minuten geschüttelt. Die organische Phase wird abgetrennt, die wässrige Phase wird zweimal mit ca. 20 ml Ethanol/Benzol-Lösung ausgeschüttelt. Die gesammelte organische Phase wird im Rotationsverdampfer bis fast zur Trockene eingedampft. Der Rückstand wird mit 5 ml tert. Butanol gelöst, mit Wasser auf 50 ml aufgefüllt und kräftig durchmischt. 2 ml der filtrierten, weitgehend klaren Lösung werden zur Lecithinbestimmung eingesetzt, die, wie in Beispiel 1 beschrieben, durchgeführt wird.

*Beispiel 3*

*Bestimmung von Lecithin in Eierlikör*

In einen 100-ml-Messkolben werden 2 g Eierlikör eingewogen und mit ca. 10 ml tert. Butanol gelöst. Mit Wasser wird auf 100 ml aufgefüllt und gemischt. 0,2 ml dieser Lösung werden zur Lecithinbestimmung eingesetzt, die, wie in Beispiel 1 angegeben, durchgeführt wird.

*Beispiel 4*

*Bestimmung von Lecithin in Backwaren*

2 g einer Backwarenprobe werden zerkleinert und in einen 100-ml-Scheidetrichter übergeführt. Es werden 30 ml Ethanol/Benzol-Lösung (1 + 1, v/v) und 50 ml Wasser hinzugegeben. Das Gemisch wird 5 Minuten kräftig geschüttelt. Die organische Phase wird abgetrennt. Die wässrige Phase wird zweimal mit je 10 ml Ethanol/Benzol-Lösung ausgeschüttelt. Die organischen Phasen werden vereinigt, filtriert und am Rotationsverdampfer nahezu zur Trockene eingedampft, wobei der Rotationsverdampfer bis auf höchstens 45°C erhitzt werden darf. Der Rückstand wird in 10 ml tert. Butanol gelöst, mit Wasser auf 50 ml aufgefüllt und kräftig durchmischt. 1 ml der filtrierten Lösung werden zur Lecithinbestimmung eingesetzt, die, wie in Beispiel 1 beschrieben, durchgeführt wird.

**Patentansprüche**

1. Verfahren zur Bestimmung von Lecithin in einer Probe, bei dem Lecithin mit Hilfe von Phospholipase-C und alkalischer Phosphatase zu Cholin gespalten, dieses in Gegenwart von Adenosintriphosphat mit Hilfe von Cholinkinase wieder zu Phosphorylcholin umgesetzt und das dabei entstehende Adenosindiphosphat nach bekannten Methoden gemessen wird, dadurch gekennzeichnet, dass die Spaltung des Lecithins in Gegenwart eines Borat/Borax-Puffers und eines nicht-ionischen Detergens auf der Basis eines Kondensationsproduktes von Polyethylenglykol mit Isooctylphenol durchgeführt wird.

2. Verfahren gemäss Anspruch 1, dadurch gekennzeichnet, dass ein Borat/Borax-Puffer, pH 7,0 bis 8,5, in einer Konzentration von 0,05-0,5 mol/l Pufferlösung und das Detergens in einer Konzentration von 0,5-5,0 g/l Pufferlösung zugesetzt werden.

3. Verfahren gemäss einem der Ansprüche 1 und 2, dadurch gekennzeichnet, dass 0,07-0,25 mol/l Pufferlösung Borat/Borax-Puffer und 12,0-3,0 g/l Pufferlösung Detergens eingesetzt werden.

4. Verfahren gemäss einem der Ansprüche 1 bis 3, dadurch gekennzeichnet, dass als Detergens Tinovetin JU® verwendet wird.

5. Reagens zur Durchführung des Verfahrens gemäss den Ansprüchen 1 bis 4, gekennzeichnet durch folgende Bestandteile:

a) einer Pufferlösung mit
0,05-0,5 mol/l Borat/Borax-Puffer, pH 7,0 bis 8,5
2-20 mmol/l Magnesiumionen
0,5-5,0 g/l Detergens

b) einer Enzymsuspension A mit
40-200 U/ml Phospholipase-C
20-200 U/ml alkalische Phosphatase

c) einer Coenzym-Lösung mit
2,5-6,0 mmol/l NADH
10-30 mmol/l ATP
3-10 mmol/l Phosphoenolpyruvat
30-100 mmol/l Glukose

d) einer Enzymsuspension B mit
150-1000 U/ml Pyruvatkinase
150-1000 U/ml Lactatdehydrogenase

e) einer Lösung mit
2-10 U/ml Cholinkinase.

6. Reagens gemäss Anspruch 5, gekennzeichnet durch folgende Bestandteile:

a) einer Pufferlösung mit
0,2 mol/l Borat/Borax-Puffer, pH 7,8-8,2
10 mmol/l Magnesiumsulfat
2,5 g/l Tinovetin JU®

b) einer Enzymsuspension A mit
80 U/ml Phospholipase-C
100 U/ml alkalische Phosphatase

c) einer Coenzym-Lösung mit
4,0 mmol/l NADH
20 mmol/l ATP
7 mmol/l Phosphoenolpyruvat
56 mmol/l Glukose

d) einer Enzymsuspension B mit
300 U/ml Pyruvatkinase
300 U/ml Lactatdehydrogenase

e) einer Lösung mit
2,5 U/ml Cholinkinase.

7. Reagens zur Spaltung des Lecithins, gekennzeichnet durch einen Gehalt an

2-10 U/ml Phospholipase-C
1-10 U/ml alkalische Phosphatase
0,05-0,5 mol/l Borat/Borax-Puffer, pH 7,0-8,5
2-20 mmol/l Magnesiumionen
0,5-5,0 g/l Detergens auf der Basis eines Kondensationsproduktes von Polyethylenglykol mit Isooctylphenol.

8. Reagens gemäss Anspruch 7, gekennzeichnet durch einen Gehalt an

4 U/ml Phospholipase-C
5 U/ml alkalische Phosphatase-C
0,2 mol/l Borat/Borax-Puffer, pH 7,8-8,2
10 mmol/l Magnesiumsulfat
2,5 g/l Tinovetin JU®.

## Claims

1. Process for the determination of lecithin in a sample, in which lecithin is split with the help of phospholipase-C and alkaline phosphatase to choline, this is reacted in the presence of adenosine triphosphate with the help of choline kinase again to give phosphoryl-choline and the thereby formed adenosine diphosphate is measured according to known methods, characterised in that the splitting of the lecithin is carried out in the presence of a borate/borax buffer and of a non-ionic detergent based on a condensation product of polyethyleneglycol with isooctylphenol.

2. Process according to claim 1, characterised in that a borate/borax buffer, pH 7.0-8.5, is added in a concentration of 0.05-0.5 mole/l. of buffer solution and the detergent in a concentration of 0.5-5.0 g./l. of buffer solution.

3. Process according to one of claims 1 and 2, characterised in that borate/borax buffer is added at 0.07-0.25 mole/l. of buffer solution and detergent at 1.0-3.0 g./l. of buffer solution.

4. Process according to one of claims 1 to 3, characterised in that Tinovetin JU® is used as detergent.

5. Reagent for the carrying out of the process according to claims 1 to 4, characterised by the following components:

a) a buffer solution with
     0.05-0.5 mole/l. borate/borax buffer, pH 7.0 to 8.5
     2-20 mmole/l. magnesium ions
     0.5-5.0 g./l. detergent

b) an enzyme suspension A with
     40-200 U/ml. phospholipase-C
     20-200 U/ml. phosphatase

c) a coenzyme solution with
     2.5-6.0 mmole/l. NADH
     10-30 mmole/l. ATP
     3-10 mmole/l. phosphoenol pyruvate
     30-100 mmole/l. glucose

d) an enzyme suspension B with
     150-1000 U/ml. pyruvate kinase
     150-1000 U/ml. lactate dehydrogenase

e) a solution with
     2-10 U/ml. choline kinase.

6. Reagent according to claim 5, characterised by the following components:

a) a buffer solution with
     0.2 mole/l. borate/borax buffer, pH 7.8-8.2
     10 mmole/l. magnesium sulphate
     2.5 g./l. Tinovetin JU®

b) an enzyme suspension A with
     80 U/ml. phospholipase-C
     100 U/ml. alkaline phosphatase

c) a coenzyme solution with
     4.0 mmole/l.NADH
     20 mmole/l. ATP
     7 mmole/l. phosphoenol pyruvate
     56 mmole/l. glucose

d) an enzyme suspension B with
     300 U/ml. pyruvate kinase
     300 U/ml. lactate dehydrogenase

e) a solution with
     2.5 U/ml. choline kinase.

7. Reagent for splitting lecithin, characterised by a content of:

2-10 U/ml. phospholiphase-C
1-10 U/ml. alkaline phosphatase
0.05-0.5 mole/l. borate/borax buffer, pH 7.0-8.5
2-20 mmole/l. magnesium ions
0.5-5.0 g./l. of detergent based on a condensation product of polyethylene glycol with isooctylphenol.

8. Reagent according to claim 7, characterised by a content of:

4 U/ml. phospholipase-C
5 U/ml. alkaline phosphatase
0.2 mole/l. borate/borax buffer, pH 7.8-8.2
10 mmole/l. magnesium sulphate
2.5 g./l. Tinovetin JU®.

## Revendications

1. Procédé pour le dosage de la lécithine dans un échantillon, dans lequel la lécithine est décomposée en choline au moyen de phospholipase-C et de phosphatase alcaline, la choline est de nouveau transformée en phosphoryl-choline à l'aide de choline-kinase en présence de triphosphate d'adénosine, le diphosphate d'adénosine formé au cours de cette transformation étant dosé selon une méthode connue, caractérisé en ce que la décomposition de la lécithine est réalisée en présence d'un tampon borate/borax et d'un détergent à base d'un produit de condensation de polyéthylèneglycol et d'iso-octylphénol.

2. Procédé selon la revendication 1, caractérisé en ce qu'un tampon borate/borax, pH 7,0 à 8,5 est ajouté en une concentration comprise entre 0,05 et 0,5 mole/l de solution tampon et le détergent en une concentration comprise entre 0,5 et 5,0 g/l de solution tampon.

3. Procédé selon l'une des revendications 1 et 2, caractérisé en ce qu'on met en oeuvre entre 0,07 et 0,25 mole de tampon borate/borax par litre de solution tampon et entre 1,0 et 3,0 g de détergent par litre de solution tampon.

4. Procédé selon l'une des revendications 1 à 3, caractérisé en ce qu'on utilise comme détergent du Tinovetin JU (marque déposée).

5. Réactif pour la mise en oeuvre du procédé selon les revendications 1 à 4, caractérisé par les ingrédients suivants:

a) une solution tampon contenant
     entre 0,05 et 0,5 mole/l de tampon borate/borax, pH 7,0 à 8,5
     entre 2 et 20 mmoles/l d'ions magnésium
     entre 0,5 et 5,0 g/l de détergent

b) une suspension d'enzymes A contenant
     entre 40 et 200 U/ml de phospholipase-C
     entre 20 et 200 U/ml de phosphatase alcaline

c) une solution de co-enzymes contenant
entre 2,5 et 6,0 mmoles/l de NADH
entre 10 et 30 mmoles/l d'ATP
entre 3 et 10 mmoles/l de phospho-énolpyruvate
entre 30 et 100 mmoles/l de glucose

d) une suspension d'enzymes B contenant
entre 150 et 1000 U/ml de pyruvate-kinase
entre 150 et 1000 U/ml de lacticodéshydrogénase

e) une solution contenant
entre 2 et 10 U/ml de choline-kinase.

6. Réactif selon la revendication 5, caractérisé par les ingrédients suivants:

a) une solution tampon contenant
0,2 mole/l de tampon borate/borax, pH 7,8 à 8,2
10 mmoles/l de sulfate de magnésium
2,5 g/l de Tinovetin JU (marque déposée)

b) une suspension d'enzymes A contenant
80 U/ml de phospholipase-C
100 U/ml de phosphatase alcaline

c) une solution de co-enzymes contenant
4,0 mmoles/l de NADH
20 mmoles/l d'ATP

7 mmoles/l de phospho-énolpyruvate
56 mmoles de glucose

d) une suspension d'enzymes B contenant
300 U/ml de pyruvate-kinase
300 U/ml de lacticodéshydrogénase

e) une solution contenant
2,5 U/ml de choline-kinase.

7. Réactif pour la décomposition de la lécithine, caractérisé parce qu'il contient:
entre 2 et 10 U/ml de phospholipase-C
entre 1 et 10 U/ml de phosphatase alcaline
entre 0,05 et 0,5 mole/l de tampon borate/borax, pH 7,0 à 8,5
entre 2 et 20 mmoles/l d'ions magnésium
entre 0,5 et 5,0 g/l de détergent à base d'un produit de condensation de polyéthylèneglycol et d'iso-octylphénol.

8. Réactif selon la revendication 7, caractérisé en ce qu'il contient:
4 U/ml de phospholipase-C
5 U/ml de phosphatase alcaline
0,2 mole/l de tampon borate/borax, pH 7,8 à 8,2
10 mmole/l de sulfate de magnésium
2,5 g/l Tinovetin JU (marque déposée).